# EUROPEAN PATENT APPLICATION

(11) **EP 4 133 988 A1**
(43) Date of publication of application: **15.02.2023**
(21) Application number: 21784240.0
(22) Date of filing: 15.03.2021
(51) Int. Cl.: A61B 1/00, A61B 1/045

(54) **ENDOSCOPE INSERTION ASSISTANCE DEVICE, METHOD, AND NON-TEMPORARY COMPUTER-READABLE MEDIUM HAVING PROGRAM STORED THEREIN**

(30) Priority: 09.04.2020 JP 2020070370
(71) Applicant: NEC Corporation, 108-8001 Tokyo (JP); Olympus Corporation, Hachioji-shi Tokyo 192-8507 (JP)
(72) Inventor: KAMIJO Kenichi, Tokyo 108-8001 (JP); NAKAO Yusuke, Tokyo 108-8001 (JP); MANO Mieko, Tokyo 108-8001 (JP); IKEDA Yuichi, Hachioji-shi, Tokyo 192-8507 (JP); FUJITA Hiromasa, Hachioji-shi, Tokyo 192-8507 (JP)
(74) Representative: Betten & Resch
(86) International application number: PCT/JP2021/010362
(87) International publication number: WO 2021/205818

(57) **Abstract**

It is an object of the present invention to provide an endoscope insertion assistance apparatus, a method and a program that allow an operator of an endoscope to easily grasp an insertion situation of the endoscope to thereby conduct effective insertion assistance. An endoscope insertion assistance apparatus (100) according to the present invention includes an acquisition unit (110) that acquires shape data to identify an insertion shape of an endoscope inserted into a lumen, an estimation unit (120) that estimates any one of a plurality of shape categories for the insertion shape from the shape data and an output unit (130) that outputs display information in chronological order for the estimated shape category.

## Description

### Technical Field

The present invention relates to an endoscope insertion assistance apparatus, a method and a program, and more specifically, to an endoscope insertion assistance apparatus, a method and a program for assisting insertion of an endoscope.

### Background Art

Insertion of an endoscope to observe inside a body such as a large intestine or small intestine has become common. On such occasion, an operator (medical doctor) needs to understand what shape an insertion portion of the endoscope takes in the body.

Thus, Patent Literature 1 discloses a technique related to an analysis apparatus that analyzes an insertion shape of an endoscope. The analysis apparatus acquires insertion shape data from an endoscope insertion shape observation apparatus and detects the shape of the insertion portion of the endoscope from the acquired insertion shape data. The analysis apparatus analyzes and obtains a specific part or a specific portion of the detected shape and classifies a static shape of the insertion portion of the endoscope into patterns based on an analysis result thereof. The analysis apparatus then displays the classified patterns on a screen.

### Citation List

### Patent Literature

Patent Literature 1: Japanese Unexamined Patent Application Publication No.

### Summary of Invention

### Technical Problem

Here, actual endoscopy needs to be conducted by a medical doctor skilled in operation and there is a problem that a high degree of difficulty is involved in the operation of the endoscope. That is, a mechanism that provides insertion assistance for the operator operating the endoscope is required. Note that there is room for improvement in insertion assistance in the technique related to aforementioned Patent Literature 1.

The present disclosure has been implemented to solve the above-described problem, and it is an object of the present disclosure to provide an endoscope insertion assistance apparatus, a method and a program that allow the operator of the endoscope to easily grasp an insertion situation of the endoscope and conduct effective insertion assistance.

### Solution to Problem

An endoscope insertion assistance apparatus according to a first aspect of the present disclosure includes an acquisition unit that acquires shape data to identify an insertion shape of an endoscope inserted into a lumen, an estimation unit that estimates any one of a plurality of shape categories for the insertion shape from the shape data and an output unit that outputs display information in chronological order for the estimated shape category.

An endoscope insertion assistance method according to a second aspect of the present disclosure includes a computer acquiring shape data to identify an insertion shape of an endoscope inserted into a lumen, estimating any one of a plurality of shape categories for the insertion shape from the shape data and outputting display information in chronological order for the estimated shape category.

An endoscope insertion assistance program according to a third aspect of the present disclosure causes a computer to execute an acquisition process of acquiring shape data to identify an insertion shape of an endoscope inserted into a lumen, an estimation process of estimating any one of a plurality of shape categories for the insertion shape from the shape data and an output process of outputting display information in chronological order for the estimated shape category.

### Advantageous Effects of Invention

According to the present disclosure, it is possible to provide an endoscope insertion assistance apparatus, a method and a program that allow the operator of the endoscope to easily grasp an insertion situation of the endoscope and thereby conduct effective insertion assistance.

### Brief Description of Drawings

Fig. 1 is a block diagram illustrating a configuration of an endoscope insertion assistance apparatus according to a first example embodiment;
Fig. 2 is a flowchart illustrating a flow of an endoscope insertion assistance method according to the first example embodiment;
Fig. 3 is a block diagram illustrating an overall configuration of an endoscope insertion assistance system according to a second example embodiment;
Fig. 4 is a block diagram illustrating a configuration of an endoscope insertion assistance apparatus according to the second example embodiment;
Fig. 5 is a diagram illustrating an example of shape data according to the second example embodiment;
Fig. 6 is a diagram illustrating an example of an endoscope image according to the second example embodiment;
Fig. 7 is a diagram illustrating an example of display information of chronological transition of a shape category according to the second example embodiment;
Fig. 8 is a flowchart illustrating a flow of the endoscope insertion assistance method according to the second example embodiment;
Fig. 9 is a block diagram illustrating a configuration of an endoscope insertion assistance apparatus according to a third example embodiment;
Fig. 10 is a flowchart illustrating a flow of a comparison process according to the third example embodiment;
Fig. 11 is a diagram illustrating an example of comparison results of chronological transition of a shape category according to the third example embodiment;
Fig. 12 is a block diagram illustrating a configuration of an endoscope insertion assistance apparatus according to a fourth example embodiment; and
Fig. 13 is a flowchart illustrating a flow of a search process according to the fourth example embodiment.

### Example Embodiment

Hereinafter, example embodiments of the present disclosure will be described in detail with reference to the accompanying drawings. In the respective drawings, identical or corresponding elements are assigned identical reference numerals and overlapping description will be omitted as required for clarification of description.

### <First Example Embodiment>

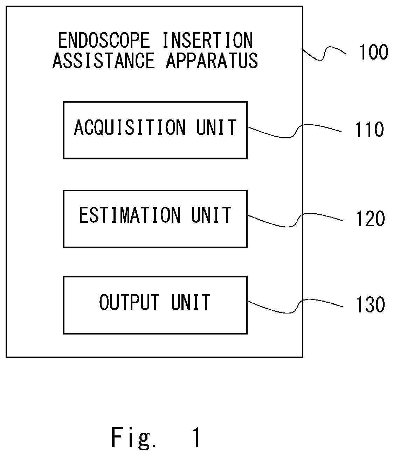

Fig. 1 is a block diagram illustrating a configuration of an endoscope insertion assistance apparatus 100 according to a first example embodiment. The endoscope insertion assistance apparatus 100 is an information processing apparatus for assisting an examiner (medical doctor) who performs examination, in operation of inserting an endoscope into a body of an examinee (subject) using the endoscope. The endoscope insertion assistance apparatus 100 is provided with an acquisition unit 110, an estimation unit 120 and an output unit 130.

The acquisition unit 110 acquires shape data for identifying an insertion shape of the endoscope inserted into a body cavity (lumen). Here, the shape data is information for identifying a shape of a fiber cable included in the endoscope when the endoscope is inserted into the body cavity, and is, for example, image data or the like for two-dimensionally expressing three-dimensional positional coordinates and shape. The acquisition unit 110 may also receive insertion shape data generated by a shape processing apparatus of the endoscope insertion shape observation apparatus as shown, for example, in Patent Literature 1 as the shape data.

The estimation unit 120 estimates any one of a plurality of shape categories for insertion shapes from the shape data. Here, the shape category is a pattern defined by grouping a plurality of shapes with similar insertion shape characteristics. At least two or more shape categories are defined, and the estimation unit 120 performs estimations by classifying the shape data acquired by the acquisition unit 110 into any one of the plurality of shape categories. Note that, the estimation unit 120 may also perform estimations using a learned model learned from learning data labeled with a shape category on each of the plurality of pieces of shape data.

The output unit 130 outputs display information in chronological order for the estimated shape category. That is, the output unit 130 generates and outputs display information visualized in a chronological format sequentially for the plurality of estimated shape categories in accordance with the insertion situation of the endoscope.

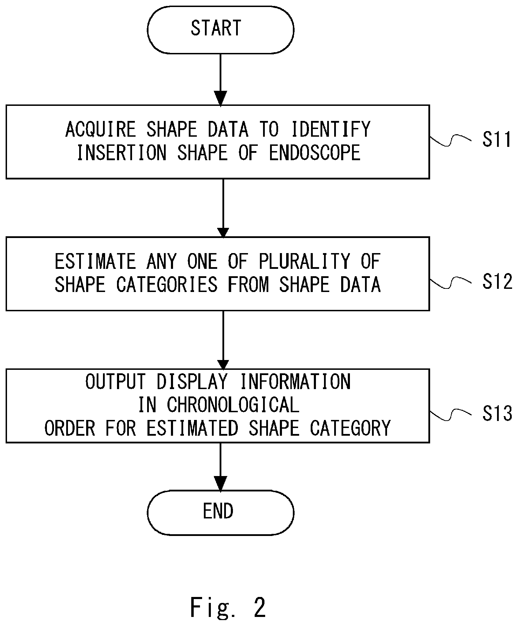

Fig. 2 is a flowchart illustrating a flow of an endoscope insertion assistance method according to the first example embodiment. First, the acquisition unit 110 acquires shape data for identifying an insertion shape of the endoscope inserted into the body cavity (S 11). Next, the estimation unit 120 estimates any one of the plurality of shape categories in the insertion shape from the shape data (S12). The output unit 130 then outputs display information for the estimated shape category in chronological order (S13).

Thus, in the present example embodiment, the acquired shape data is classified (estimated) into predetermined shape categories indicating features of the insertion shape, the acquired and estimated shape categories are sequentially output as display information in a chronological format and presented to the operator of the endoscope. Therefore, the operator of the endoscope can easily grasp the insertion situation of the endoscope. Therefore, according to the present example embodiment, it is possible to perform effective insertion assistance for the endoscope.

Note that, the endoscope insertion assistance apparatus 100 is provided with a processor, a memory and a storage apparatus as not shown components. The storage apparatus stores a computer program in which the processes of the endoscope insertion assistance method according to the present example embodiment are implemented. The processor reads the computer program from the storage apparatus into the memory and executes the computer program. In this way, the processor implements the functions of the acquisition unit 110, the estimation unit 120 and the output unit 130.

Alternatively, the acquisition unit 110, the estimation unit 120 and the output unit 130 may also be implemented by dedicated hardware. Some or all components of each apparatus may be implemented by a general-purpose or dedicated circuitry, a processor or a combination thereof. The components may be constructed of a single chip or a plurality of chips connected via a bus. Some or all components of each apparatus may also be implemented by a combination of the aforementioned circuitry and the program. A CPU (central processing unit), GPU (graphics processing unit), FPGA (field-programmable gate array) or the like can be used as the processor.

When some or all components of the endoscope insertion assistance apparatus 100 are implemented by a plurality of information processing apparatuses or circuits, the plurality of information processing apparatuses and circuits may be centrally arranged or may be distributed. For example, the information processing apparatus or circuit or the like may also be implemented in a mode in which a client server system and a cloud computing system or the like are connected via a communication network. Moreover, the function of the endoscope insertion assistance apparatus 100 may be provided in a SaaS (software as a service) format.

### <Second Example Embodiment>

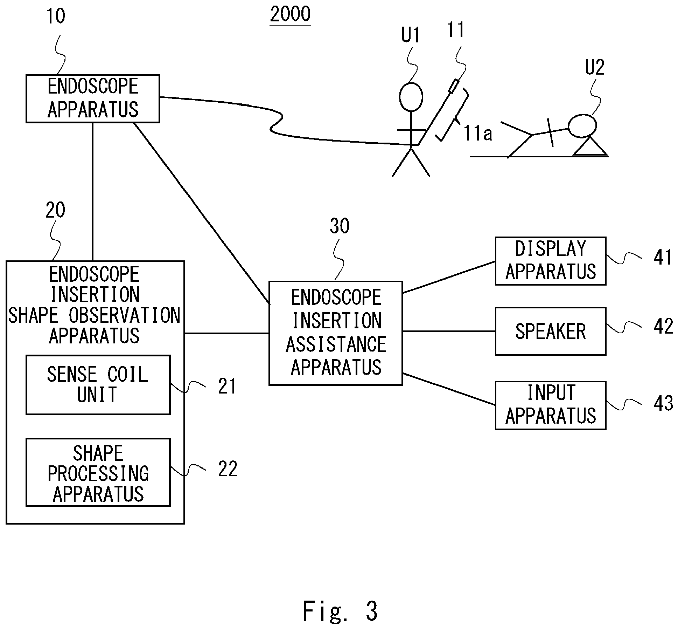

A second example embodiment is a specific example of the aforementioned first example embodiment. Fig. 3 is a block diagram illustrating an overall configuration of an endoscope insertion assistance system 2000 according to the second example embodiment. The endoscope insertion assistance system 2000 is a system that assists a medical doctor U1 in insertion of an electronic endoscope 11 when the medical doctor U1 performs an examination on an examinee U2 using the electronic endoscope 11. The endoscope insertion assistance system 2000 is provided with an endoscope apparatus 10, an endoscope insertion shape observation apparatus 20, an endoscope insertion assistance apparatus 30, a display apparatus 41, a speaker 42 and an input apparatus 43.

The endoscope apparatus 10 is provided with an electronic endoscope 11 connected to the body of the endoscope apparatus 10 via a cable. The electronic endoscope 11 includes an insertion portion 11a, which is a portion to be inserted into a body cavity of the examinee U2. The insertion portion 11a is provided with a distal end portion and a fiber cable and a light guide cable connected to the distal end portion. An end of the insertion portion 11a is connected to the proximal end portion and the body of the endoscope apparatus 10 via the above-described cable. The distal end portion is provided with an electronic image pickup device, an observation light irradiation portion and a bending portion or the like. The electronic image pickup device is equivalent to a camera of the electronic endoscope 11, and is, for example, a CCD (charge coupled device). The irradiation portion radiates observation light from the light guide cable. The bending portion causes the distal end portion to bend in response to a control signal from an operation portion of the proximal end portion.

The fiber cable and the light guide cable are also connected to the body of the endoscope apparatus 10. The fiber cable transmits/receives various signals to/from the body of the endoscope apparatus 10, and particularly transmits an image (endoscope image) captured by the electronic image pickup device to the body of the endoscope apparatus 10. The body of the endoscope apparatus 10 outputs the endoscope image to the endoscope insertion assistance apparatus 30. The light guide cable guides light from a light source of the endoscope apparatus 10 to the irradiation portion.

Here, the insertion portion 11a is provided with source coils (not shown) at a plurality of locations. The source coils are arranged, for example, at a predetermined interval. The source coils generate magnetic fields in response to drive signals from the endoscope insertion shape observation apparatus 20. Therefore, the electronic endoscope 11 needs only to incorporate magnetic coils to identify shapes at a plurality of locations of the insertion portion 11a. Note that since publicly known components can be used for the other components of the endoscope apparatus 10 and the electronic endoscope 11, illustrations and descriptions of the other components will be omitted. Alternatively, an appliance with a built-in coil inserted into the endoscope can also be used as the magnetic coil (not the one incorporated in the endoscope).

The endoscope insertion shape observation apparatus 20 is provided with at least a sense coil unit 21 and a shape processing apparatus 22. The sense coil unit 21 is a unit that detects a magnetic field generated from the plurality of source coils provided in the insertion portion 11a of the electronic endoscope 11.

The shape processing apparatus 22 outputs a drive signal to the electronic endoscope 11. When the examinee U2 wears a magnetic coil, the shape processing apparatus 22 is also supposed to output a drive signal to the magnetic coil. In this case, the sense coil unit 21 further detects the magnetic field generated from the magnetic coil attached to the examinee U2.

The shape processing apparatus 22 obtains a three-dimensional shape of the insertion portion 11a based on the magnetic field detected by the sense coil unit 21. For example, the shape processing apparatus 22 calculates three-dimensional coordinates of each source coil based on the detected magnetic field and uses a set of three-dimensional coordinates as the shape data. Alternatively, the shape processing apparatus 22 generates image data resulting from projecting the calculated three-dimensional coordinates to two-dimensional coordinates and uses the image data as the shape data.

Furthermore, when the examinee U2 wears the magnetic coil, the shape processing apparatus 22 may obtain a posture of the examinee U2 based on the detected magnetic field. More specifically, the shape processing apparatus 22 calculates three-dimensional coordinates indicating positions relative to the source coil for the magnetic coil worn by the examinee U2 and uses the three-dimensional coordinates as the posture data.

The shape processing apparatus 22 outputs the shape data and the posture data to the endoscope insertion assistance apparatus 30. Note that since publicly known components can be used as the other components of the endoscope insertion shape observation apparatus 20, illustrations and descriptions of the other components will be omitted. For example, the components described in aforementioned Patent Literature 1 may be used for the endoscope apparatus 10 and the endoscope insertion shape observation apparatus 20.

The endoscope insertion assistance apparatus 30 is connected to the endoscope apparatus 10, the endoscope insertion shape observation apparatus 20, the display apparatus 41, the speaker 42 and the input apparatus 43. The endoscope insertion assistance apparatus 30 is an example of the aforementioned endoscope insertion assistance apparatus 100. The endoscope insertion assistance apparatus 30 acquires an endoscope image from the endoscope apparatus 10 and acquires the shape data and the posture data from the endoscope insertion shape observation apparatus 20.

The endoscope insertion assistance apparatus 30 estimates a shape category of the insertion shape of the insertion portion 11a of the electronic endoscope 11 based on the acquired shape data, endoscope image and posture data and records the estimated shape category together with time information. The endoscope insertion assistance apparatus 30 generates display information to display the recorded shape category in a chronological format and outputs the display information to the display apparatus 41. The endoscope insertion assistance apparatus 30 outputs speech corresponding to the estimated shape category to the speaker 42.

The display apparatus 41 displays the display information received from the endoscope insertion assistance apparatus 30 on the screen. The speaker 42 outputs the speech received from the endoscope insertion assistance apparatus 30. The input apparatus 43 receives an input operation from an examiner (operator) such as the medical doctor U1 or an examination assistant and outputs a control signal corresponding to the input operation to the endoscope insertion assistance apparatus 30. The input apparatus 43 is, for example, a mouse or a keyboard. Note that when the display apparatus 41 is a touch panel, the display apparatus 41 and the input apparatus 43 are integrated. Note that some or all of the display apparatus 41, the speaker 42 and the input apparatus 43 may be incorporated in the endoscope insertion assistance apparatus 30.

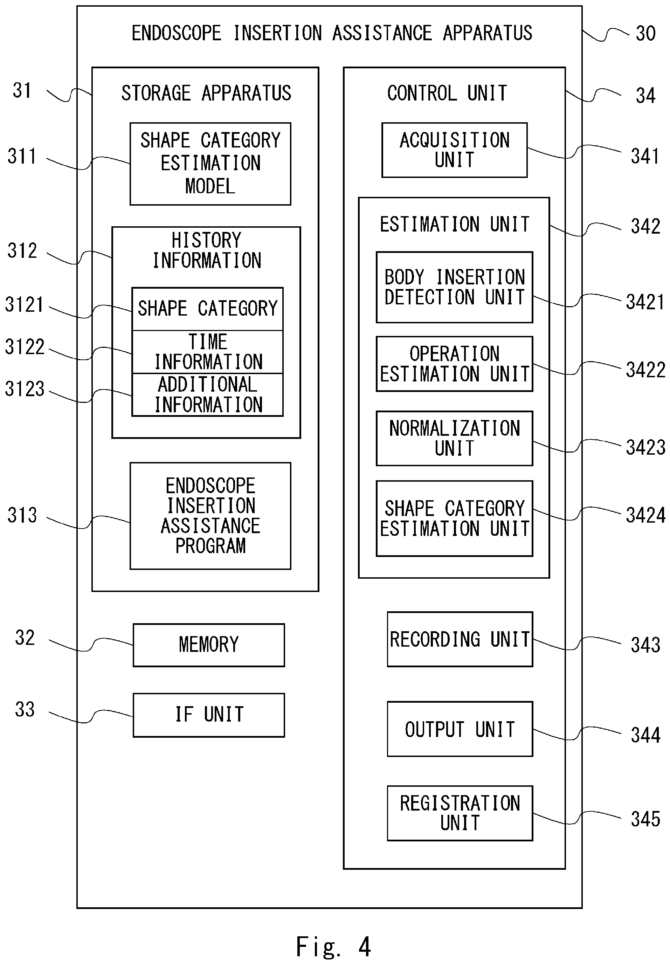

Fig. 4 is a block diagram illustrating a configuration of the endoscope insertion assistance apparatus 30 according to the second example embodiment. The endoscope insertion assistance apparatus 30 is provided with a storage apparatus 31, a memory 32, an IF (interface) unit 33 and a control unit 34. The storage apparatus 31 is a storage apparatus such as a hard disk or a flash memory. The storage apparatus 31 stores a shape category estimation model 311, history information 312, and an endoscope insertion assistance program 313.

The shape category estimation model 311 is a program module or a model expression in which a logic for estimating a shape category from shape data (or shape data, which is normalized data) is implemented. The shape category estimation model 311 is a model that receives a set of three-dimensional coordinates or image data, which is shape data, as input, estimates a shape category to which the shape of the insertion portion 11a represented by the shape data is more likely to correspond and outputs a shape category as an estimation result. Note that the shape category estimation model 311 can be said to be a learned model learned in advance from learning data labeled with "shape category" for each of the plurality of pieces of shape data.

Here, examples of the shape category include "straight line," "small curve," "large curve," "abdominal protrusion," but the shape category is not limited to these examples. The shape category does not indicate the shape of the insertion portion 11a alone, but includes a condition of the body cavity (shape of an organ). For example, the shape category may be treated as a different shape category depending on whether the bending portion of the distal end portion of the insertion portion 11a is caught in folds of a lumen or the bending portion fails to catch on folds of a lumen. That is, the shape category may represent the shape of the insertion portion 11a and may be classified with the shape of an organ to be examined taken into account. In that case, the shape category estimation model 311 further receives operation contents of the electronic endoscope 11, which will be described later, as input.

The history information 312 is information in which a shape category 3121, time information 3122 and additional information 3123 are associated with one another. Note that the additional information 3123 is not essential to the history information 312. The shape category 3121 is information representing each of the aforementioned categories, for example, identification information or a character string. The time information 3122 is time at which the shape data is acquired, time at which the shape category is estimated and the like. The additional information 3123 is additional information on an estimated shape category. The additional information 3123 is, for example, a region in the body cavity, speech pronounced by the medical doctor U1 and the like. With the history information 312, it is possible to efficiently generate transition information between shape categories, which will be described later, in chronological order.

The endoscope insertion assistance program 313 is a computer program in which the processes of the endoscope insertion assistance method according to the present example embodiment are implemented.

The memory 32 is a volatile storage apparatus such as RAM (random access memory) and is a storage region for temporarily retaining information during operation of the control unit 34. The IF unit 33 is an interface that provides inputs/outputs for the endoscope insertion assistance apparatus 30 to/from an external device. For example, the IF unit 33 receives an operation of the user via the input apparatus 43 and outputs the received operation contents to the control unit 34. The IF unit 33 receives an endoscope image or the like from the endoscope apparatus 10, receives the shape data and the posture data from the endoscope insertion shape observation apparatus 20, stores the received shape data and posture data such as the endoscope image in the memory 32 and notifies the control unit 34 of such data. The IF unit 33 provides outputs for the display apparatus 41 or the speaker 42 in response to an instruction from the control unit 34.

The control unit 34 is a processor, that is, a control apparatus that controls each component of the endoscope insertion assistance apparatus 30. The control unit 34 reads the endoscope insertion assistance program 313 from the storage apparatus 31 into the memory 32 and executes the endoscope insertion assistance program 313. Thus, the control unit 34 implements the functions of an acquisition unit 341, an estimation unit 342, a recording unit 343, an output unit 344 and a registration unit 345.

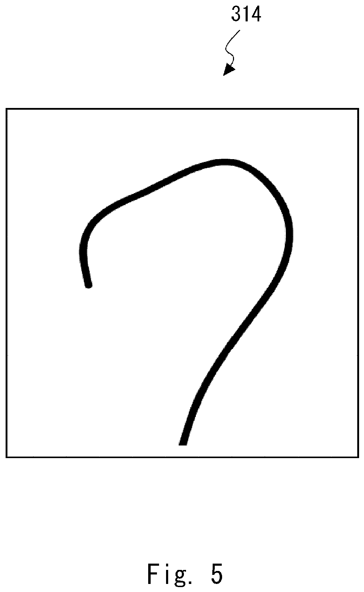

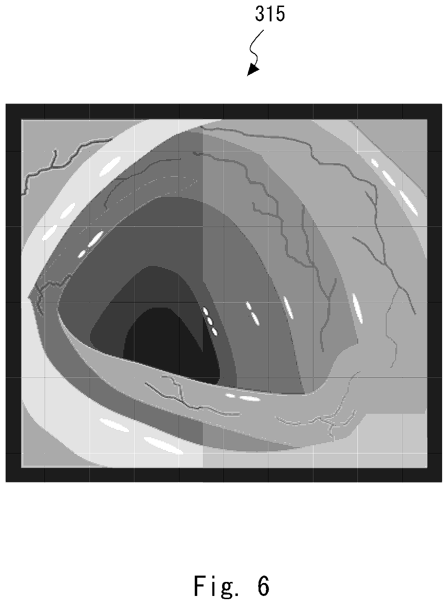

The acquisition unit 341 is an example of the aforementioned acquisition unit 110. The acquisition unit 341 acquires an endoscope image from the endoscope apparatus 10 via the IF unit 33 and acquires the shape data and the posture data from the endoscope insertion shape observation apparatus 20 via the IF unit 33. Fig. 5 is a diagram illustrating an example of shape data 314 (image data) according to the second example embodiment. Fig. 6 is a diagram illustrating an example of an endoscope image 315 according to the second example embodiment.

The estimation unit 342 is an example of the aforementioned estimation unit 120. The estimation unit 342 includes a body insertion detection unit 3421, an operation estimation unit 3422, a normalization unit 3423 and a shape category estimation unit 3424. The body insertion detection unit 3421 recognizes the endoscope image acquired by the acquisition unit 341 and determines whether the electronic endoscope 11 is inserted into the body (e.g., mouth, nose or anus) of the examinee U2. That is, using the endoscope image, the body insertion detection unit 3421 detects that the electronic endoscope 11 is inserted into the body. Here, the body insertion detection unit 3421 may determine the mouth, nose or anus by recognizing the endoscope image. The body insertion detection unit 3421 notifies the normalization unit 3423, the shape category estimation unit 3424 and the recording unit 343 of the detection.

The operation estimation unit 3422 estimates operation contents of the electronic endoscope 11 in the body cavity based on a change of the endoscope image and the shape data, and notifies the shape category estimation unit 3424 of the estimated operation contents. Here, the operation contents of the electronic endoscope 11 include a state in which the bending portion is bent and caught in folds of the lumen or the like. For example, normally if the electronic endoscope 11 moves back and forth in the body cavity, the endoscope image also changes. In this case, the shape data may change, but the shape category may not change. However, although the shape data is similar between a state in which the bending portion is caught in folds of the lumen and a state in which the bending portion fails to catch on folds of the lumen, it can be said that there is a significant difference in the state of the body cavity (shape of the organ). Thus, in this case, the shape category is divided. When the bending portion is caught in folds of the lumen, the position of the distal end portion of the insertion portion 11a changes, whereas surroundings of the camera at the distal end portion do not change. That is, it can be said that the endoscope image does not change so much. On the other hand, when the bending portion fails to catch on folds, the position of the distal end portion changes and the distance between the camera and the film increases, and so it can be said that the endoscope image also changes. Thus, the operation estimation unit 3422 preferably estimates the operation contents of the electronic endoscope 11 and the shape of the organ in consideration of both the change of the endoscope image and the change of the shape data. Thus, the estimation accuracy improves and it is possible to perform insertion assistance more effectively.

In response to reception of the notification of detection from the body insertion detection unit 3421, the normalization unit 3423 identifies that the latest shape data at the present time acquired by the acquisition unit 341 is an examination starting point. More specifically, the normalization unit 3423 identifies the three-dimensional coordinates of the distal end portion of the three-dimensional coordinate set of the latest shape data at the present time as the examination starting point (origin). The normalization unit 3423 transforms three-dimensional coordinates of the shape data acquired thereafter by the acquisition unit 341 into a coordinate system with the examination starting point as the origin and performs normalization. The normalization unit 3423 outputs the normalized shape data to the shape category estimation unit 3424. The accuracy of normalization thus improves.

The normalization unit 3423 may normalize the shape data based on the posture data (three-dimensional coordinates) acquired by the acquisition unit 341 and output the normalized shape data to the shape category estimation unit 3424. This further improves the accuracy of normalization.

In response to reception of the notification of detection from the body insertion detection unit 3421, the shape category estimation unit 3424 starts an estimation process of the shape category. More specifically, the shape category estimation unit 3424 inputs the normalized shape data received from the normalization unit 3423 to the shape category estimation model 311 and acquires the shape category, the estimation result, as output. That is, the shape category estimation unit 3424 estimates any one of the plurality of shape categories from the shape data acquired by the acquisition unit 341 using the aforementioned learned model. In this way, by continuing learning using the accumulated learning data, it is possible to improve the accuracy of estimation. The shape category estimation unit 3424 outputs the received shape category to the recording unit 343. Furthermore, the shape category estimation unit 3424 may input the operation contents received from the operation estimation unit 3422 together with the shape data to the shape category estimation model 311 and acquire the shape category, the estimation result, as the output. In this way, because the number of types of input data increases, the estimation accuracy can further improve.

Note that the estimation unit 342 may not include the normalization unit 3423. In that case, the shape category estimation unit 3424 inputs the shape data acquired by the acquisition unit 341 to the shape category estimation model 311 as is.

The recording unit 343 identifies an examination starting time point based on the endoscope image and records the transition information in chronological order for the estimated shape category based on the examination starting time point in the storage apparatus 31 as the history information 312. More specifically, the recording unit 343 identifies the present time as an examination starting time point in response to reception of the notification of detection from the body insertion detection unit 3421. Every time the shape category 3121 is received from the estimation unit 342 after the examination starting time point, the recording unit 343 associates the present time as time information 3122 and stores the present time in the storage apparatus 31 as the history information 312. That is, the recording unit 343 starts a recording process of the shape category in response to reception of the notification of detection from the body insertion detection unit 3421. Since the time information is associated with each shape category, the history information 312 can be said to be transition information in chronological order of the shape category.

The output unit 344 is an example of the aforementioned output unit 130. The output unit 344 reads the history information 312 one record at a time from the storage apparatus 31 and generates display information indicating transition between the plurality of estimated shape categories in chronological order. The output unit 344 outputs the generated display information to the display apparatus 41 via the IF unit 33. Here, the display information may be assumed to be expressed, for example, on a two-dimensional graph, with one axis representing time and the other axis representing the shape category. That is, the output unit 344 plots at a point on the graph corresponding to the read shape category 3121 and the associated time information 3122. The output unit 344 makes drawing so as to connect between plots at neighboring pieces of time information. Thus, transition between shape categories in chronological order is realized allowing the examiner or the like to easily grasp transition of shape categories. Note that the display information is not limited to the two-dimensional graph, but the display information can be a three-dimensional graph or information, in which transition between shape categories is started in chronological order.

Furthermore, the shape category may be subdivided into multiple tiers. For example, suppose a plurality of shape subcategories belong to a specific shape category. In this case, the shape category estimation model 311 estimates shape categories down to shape subcategories in addition to shape categories. The recording unit 343 stores, in the storage apparatus 31, the shape category 3121 and the shape subcategory estimated for the same shape data in association with the history information 312. After that, the output unit 344 plots at locations on the graph corresponding to the shape subcategory and the associated time information 3122 in the read record of the history information 312. The output unit 344 makes drawing so as to connect transition between different shape subcategories even within the same shape category with lines. This makes it possible to easily grasp more accurate transition.

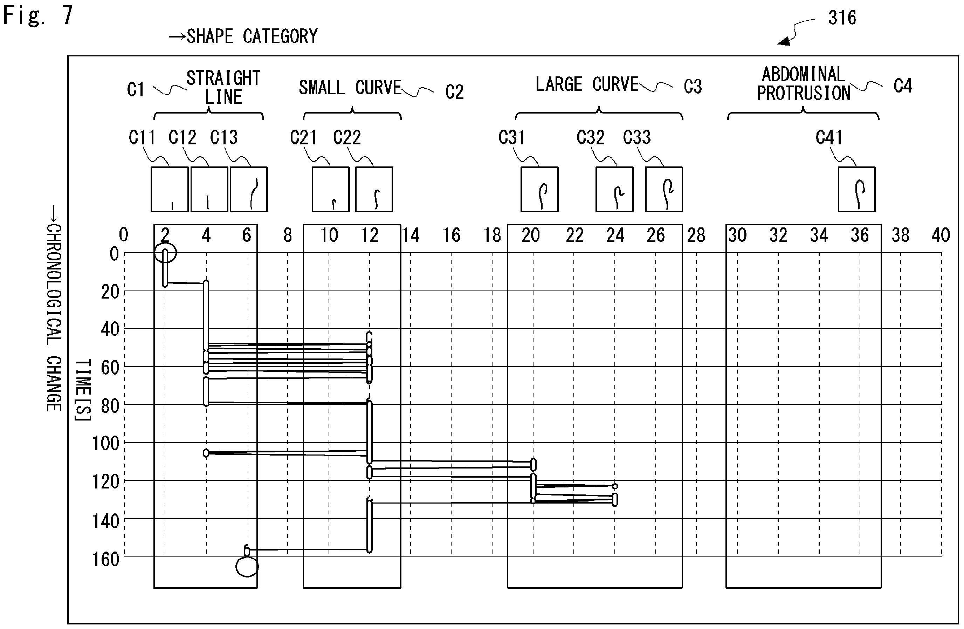

Fig. 7 is a diagram illustrating an example of display information 316 of chronological transition of a shape category according to the second example embodiment. Here, an example of a two-dimensional graph is shown with the horizontal axis representing a shape category and the vertical axis representing time information. Fig. 7 illustrates a shape category C1 "straight line," a shape category C2 "small curve," a shape category C3 "large curve", and a shape category C4 "abdominal protrusion." Fig. 7 further illustrates that three shape subcategories C11 to C13 are classified within the shape category C1, two shape subcategories C21 and C22 are classified within the shape category C2, three shape subcategories C31 to C33 are classified within the shape category C3, and one shape subcategory C41 is further classified within the shape category C4. In Fig. 7, it is possible to visually recognize transition between the shape category C1 and the shape category C2 (change of shape category) or the like. Moreover, even within the shape category C1, it is possible to visually recognize transition between the shape subcategories C11 and C12. Note that the numerical value on the horizontal axis may be a shape subcategory number.

Return to Fig. 4 for further explanation. The output unit 344 may output speech corresponding to the estimated shape category. In that case, the storage apparatus 31 is assumed to store speech data corresponding to each shape category in advance. When reading the history information 312 from the storage apparatus 31, the output unit 344 reads speech data corresponding to the shape category 3121 together. The output unit 344 outputs the read speech data to the speaker 42 via the IF unit 33. This allows the examiner to grasp the shape category of the current electronic endoscope 11 without looking at the display apparatus 41, and it is thereby possible to perform insertion assistance more effectively.

The registration unit 345 receives an input of additional information on the estimated shape category via the input apparatus 43 and the IF unit 33, further associates the additional information 3123 with the shape category 3121 and registers the additional information 3123 in the storage apparatus 31 as the history information 312. Therefore, the registration unit 345 may update the registered history information 312. When the output unit 344 reads the history information 312, the output unit 344 reads the additional information 3123 together with the shape category 3121 and can display the additional information 3123 included in the display information.

Note that the estimation unit 342 may identify a location in the body from the estimated shape category and the output unit 344 may further output the identified location. For example, when the examination target is a large intestine, examples of the internal locations include sigmoid colon, descending colon, transverse colon and ascending colon. In that case, learning data labeled with the internal locations for a combination of transitions between shape categories is created and an internal location estimation model is learned using the learning data. This allows the estimation unit 342 to input the combination of transitions between shape categories of the history information 312 to the internal location estimation model at arbitrary timing and obtain the estimated internal locations as the output. Therefore, the internal location estimation model can be used to verify the examination result after the examination. Alternatively, the estimation unit 342 can obtain estimate values of the internal locations by inputting the estimation results (combination of transitions between shape categories) from the examination starting time point to immediately before every time during the examination to the internal location estimation model. Thus, the output unit 344 outputs the estimated internal locations and can thereby grasp the internal location at which the distal end portion of the electronic endoscope 11 is currently located in real time and perform insertion assistance more effectively.

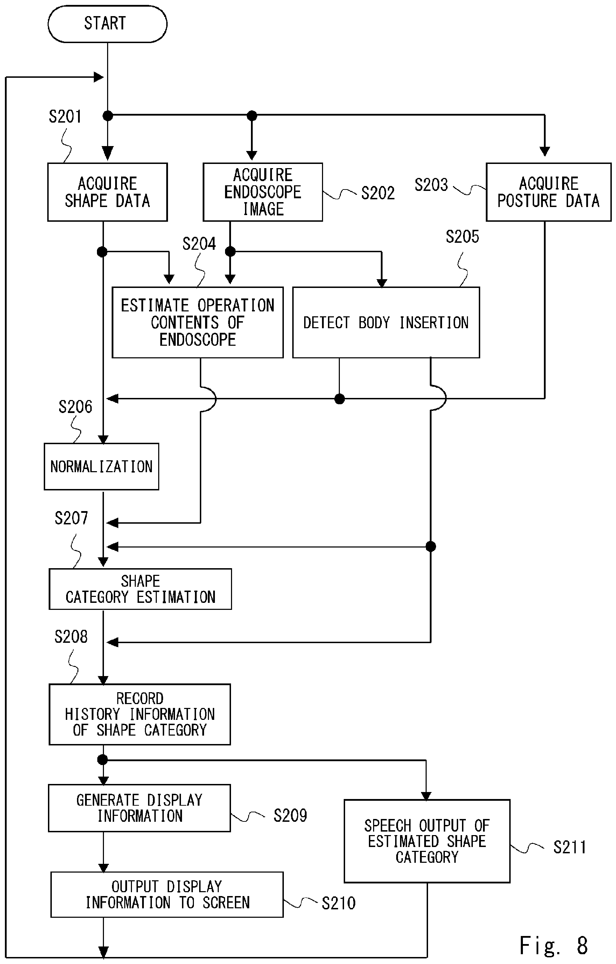

Fig. 8 is a flowchart illustrating a flow of the endoscope insertion assistance method according to the second example embodiment. First, the acquisition unit 341 acquires shape data from the endoscope insertion shape observation apparatus 20 (S201). The acquisition unit 341 outputs the acquired shape data to the normalization unit 3423 and the operation estimation unit 3422. The acquisition unit 341 acquires an endoscope image from the endoscope apparatus 10 (S202). The acquisition unit 341 outputs the acquired endoscope image to the body insertion detection unit 3421 and the operation estimation unit 3422. The acquisition unit 341 acquires posture data from the endoscope insertion shape observation apparatus 20 (S203). The acquisition unit 341 outputs the acquired posture data to the normalization unit 3423.

After steps S201 and S202, the operation estimation unit 3422 estimates the operation contents of the electronic endoscope 11 based on the change of the shape data and the endoscope image received from the acquisition unit 341 (S204). The operation estimation unit 3422 notifies the shape category estimation unit 3424 of the estimated operation contents.

After step S202, the body insertion detection unit 3421 detects that the electronic endoscope 11 has been inserted into the examinee U2 based on the endoscope image received from the acquisition unit 341 (S205). When the insertion of the electronic endoscope 11 is detected, the body insertion detection unit 3421 notifies the normalization unit 3423, the shape category estimation unit 3424 and the recording unit 343, of the notification of detection.

After steps S201, S203 and S205, the normalization unit 3423 identifies the latest shape data at the present time as the examination starting point in response to the notification of detection of body insertion from the body insertion detection unit 3421. The normalization unit 3423 performs normalization of the received shape data thereafter based on the posture data and the examination starting point acquired by the acquisition unit 341 (S206). The normalization unit 3423 outputs the normalized shape data to the shape category estimation unit 3424.

After steps S204, S205 and S206, the shape category estimation unit 3424 starts estimation of a format category in response to the notification of detection of body insertion from the body insertion detection unit 3421. More specifically, the shape category estimation unit 3424 estimates the format category from the normalized shape data received from the shape category estimation unit 3424 and the operation contents of the electronic endoscope 11 received from the operation estimation unit 3422 (S207). That is, the shape category estimation unit 3424 inputs the normalized shape data and operation contents to the shape category estimation model 311 and acquires the shape category as the estimation result. The shape category estimation unit 3424 then outputs the acquired format category to the recording unit 343.

After steps S205 and S207, the recording unit 343 identifies the present time as an examination starting time point in response to the notification of detection of body insertion from the body insertion detection unit 3421 and starts recording of the shape category on the history information. More specifically, the recording unit 343 associates the present time with the shape category 3121 received from the shape category estimation unit 3424 as time information 3122 and records (stores) the present time in the storage apparatus 31 as history information 312 (S208).

After step S208, the output unit 344 reads the history information 312, one record at a time, from the storage apparatus 31 and generates display information indicating transition between a plurality of estimated shape categories in chronological order (S209). The output unit 344 then outputs the generated display information to the screen of the display apparatus 41 (S210). After step S208, the output unit 344 outputs the speech corresponding to the estimated shape category to the speaker 42 (S211).

After steps S210 and S211, the process returns to steps S201, S202 and S203 and repeats the subsequent steps. Note that the endoscope insertion assistance method can be finished at predetermined timing. For example, when the body insertion detection unit 3421 detects that the electronic endoscope 11 has been removed from the body, the process may be finished.

In this way, the estimation unit 342 according to the present example embodiment estimates any one of the plurality of shape categories further using an endoscope image, and the estimation accuracy thereby improves compared to a case where the shape category is estimated using only the shape data. Estimation using operation contents of the electronic endoscope 11 and the posture data of the examinee U2 can further improve the estimation accuracy. By recording the estimated shape category as the history information 312, the estimated shape category can be effectively used not only during an examination but also for a post-examination analysis or the like. By displaying chronological transition between format categories on the screen, the medical doctors can easily grasp the state of the electronic endoscope 11 during an examination or the situation of the body cavity. Therefore, insertion assistance of the endoscope can be realized more effectively.

### <Third Example Embodiment>

A third example embodiment is an improvement example of the aforementioned second example embodiment. In comparison with the aforementioned endoscope insertion assistance system 2000, the endoscope insertion assistance apparatus 30 is replaced by an endoscope insertion assistance apparatus 30a in an endoscope insertion assistance system according to the third example embodiment. Therefore, illustrations thereof will be omitted and the following description will be focused on the changed parts.

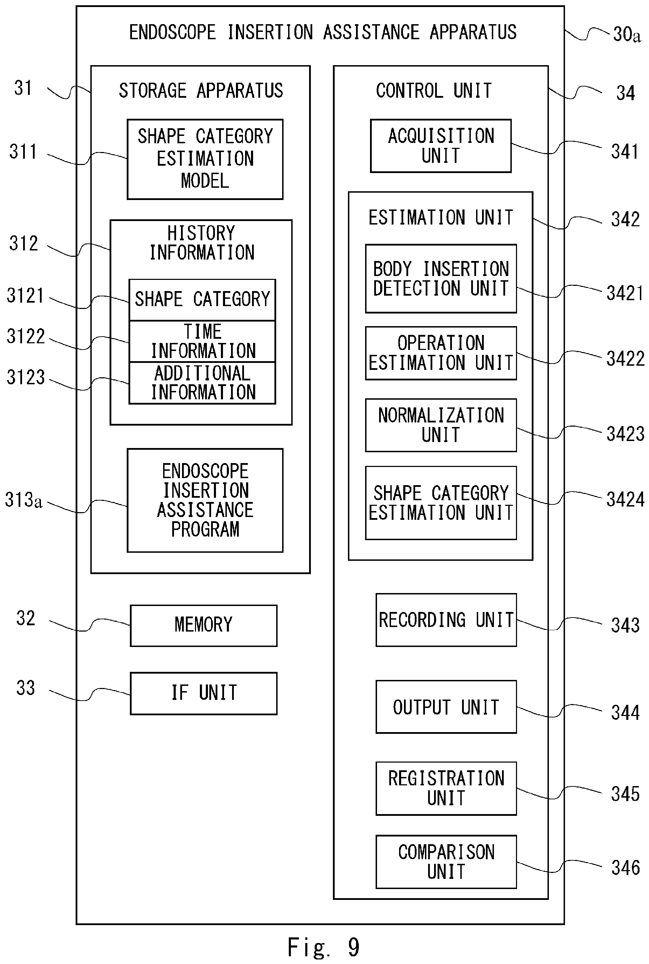

Fig. 9 is a block diagram illustrating a configuration of the endoscope insertion assistance apparatus 30a according to the third example embodiment. In comparison with the aforementioned endoscope insertion assistance apparatus 30, the endoscope insertion assistance program 313 is replaced by an endoscope insertion assistance program 313a and a comparison unit 346 is added in the endoscope insertion assistance apparatus 30a. Note that, of the control unit 34, the endoscope insertion assistance apparatus 30a needs only to include at least the acquisition unit 341, the shape category estimation unit 3424, the recording unit 343, the output unit 344 and the comparison unit 346 and may not include the other components. The endoscope insertion assistance program 313a is a computer program in which the comparison process in the endoscope insertion assistance method according to the present example embodiment is implemented.

The comparison unit 346 outputs comparison results of two or more pieces of the history information 312. This allows the medical doctors to easily compare past endoscope insertion histories and perform analysis more efficiently. By visually recognizing the comparison results, it is possible to more efficiently improve the technique of endoscope insertion operation. Note that the output by the comparison unit 346 is assumed to be output using the output unit 344. That is, the comparison unit 346 performs a comparison process on the two or more pieces of the history information 312, outputs the comparison result to the output unit 344 and the output unit 344 indicates that the comparison result is output to the display apparatus 41 or the speaker 42.

Furthermore, the comparison unit 346 may evaluate a comparison destination against a comparison source of the history information 312 and output the evaluation result as the comparison result. For example, by using history information by a skilled medical doctor as the comparison source and using history information by a relatively inexperienced medical doctor as the comparison destination, it is possible to obtain objective evaluation of the endoscope insertion operation. This allows the medical doctors to objectively grasp problems of the own endoscope insertion operation and improve the technique of endoscope insertion operation in a shorter period.

When the comparison result shows that a duration of a specific shape category is a predetermined time or more, the comparison unit 346 may output a warning. This allows the medical doctors to easily grasp a potential problem location in the endoscope insertion operation at the comparison destination.

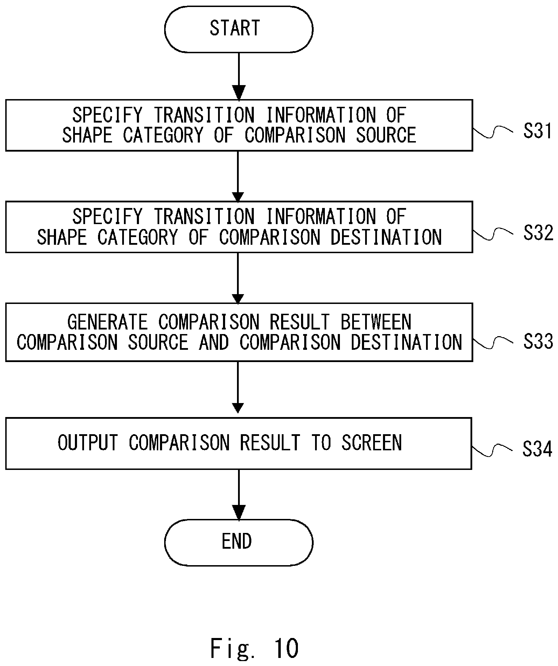

Fig. 10 is a flowchart illustrating a flow of a comparison process according to the third example embodiment. First, the comparison unit 346 receives a specification of transition information of the shape category of the comparison source via the input apparatus 43 and the IF unit 33 (S31). For example, the input apparatus 43 receives an input of date and time (examination time zone) corresponding to the past endoscopies by skilled medical doctors according to operations by the medical doctors. The input apparatus 43 transmits the received date and time information to the endoscope insertion assistance apparatus 30a. The comparison unit 346 of the endoscope insertion assistance apparatus 30a acquires a set of the shape category 3121 associated with the time information 3122 included in the time zone indicated by the received date and time information from the storage apparatus 31 and stores the set of the shape category 3121 in the memory 32 as the comparison source.

Next, the comparison unit 346 receives the specification of the transition information of the shape category at the comparison destination via the input apparatus 43 and the IF unit 33 (S32). For example, the input apparatus 43 receives inputs of date and time (examination time zone) corresponding to the past endoscopies by relatively inexperienced medical doctors according to operations by medical doctors. Hereinafter, processes similar to the process in step S31 will be performed and the comparison unit 346 of the endoscope insertion assistance apparatus 30a retains the history information acquired from the storage apparatus 31 in the memory 32 as the comparison destination.

Next, the comparison unit 346 generates comparison results between the comparison source and the comparison destination (S33). For example, the comparison unit 346 compares shape categories to which relative elapsed times from the examination starting time point correspond between the comparison source and the comparison destination, and calculates presence or absence of a difference in chronological order. Alternatively, the comparison unit 346 generates a comparison result between transition information of the comparison source and transition information of the comparison destination so as to make drawing on a two-dimensional graph by aligning their examination starting time points. Furthermore, the comparison unit 346 may evaluate the comparison destination against the comparison source. For example, the comparison unit 346 may judge the superiority of the comparison destination with respect to the comparison source and use the judgment result as the evaluation result. For example, when a duration of a specific format category is longer than a predetermined time at the comparison source, the comparison unit 346 may judge low the evaluation in the time zone. Alternatively, when a duration of a specific format category is longer than a predetermined time at the comparison source or the comparison destination, the comparison unit 346 may output a warning in the time zone with or without evaluation.

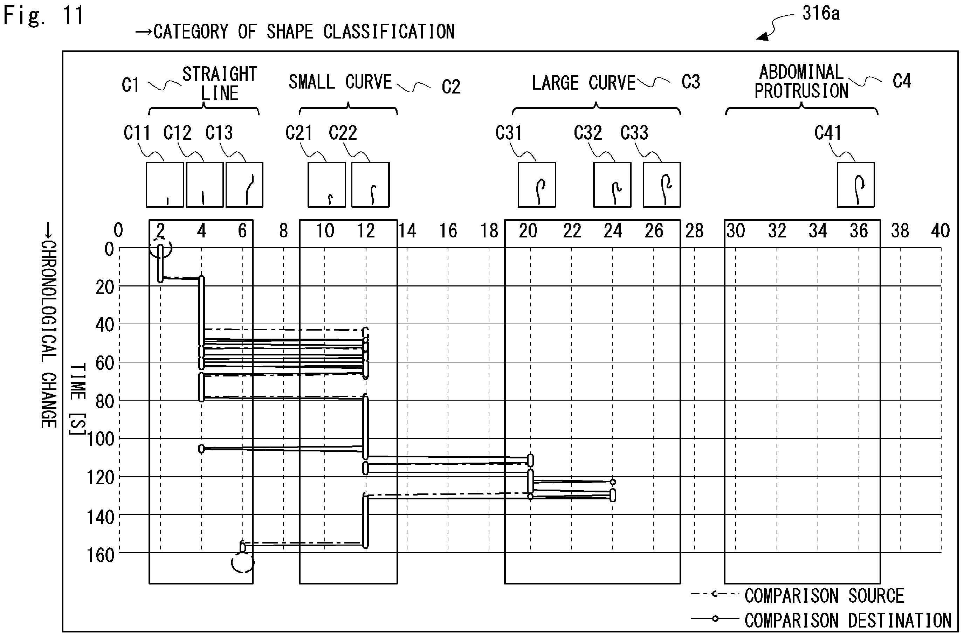

After that, the comparison unit 346 outputs the comparison result to the screen of the display apparatus 41 via the IF unit 33 (S34). Fig. 11 is a diagram illustrating an example of comparison results 316a of chronological transition of a shape category according to the third example embodiment. Here, Fig. 11 illustrates an example in which the examination starting time points are aligned so that the transition information of the comparison source and the transition information of the comparison destination overlap.

Thus, according to the present example embodiment, it is possible to effectively use the history information 312 accumulated in the second example embodiment and further promote an improvement of the endoscope insertion technique.

Note that if a medical doctor ID is associated with the history information 312, the input apparatus 43 may receive the medical doctor ID, and the comparison unit 346 may search for the history information 312 by the medical doctor ID and acquire a comparison source and a comparison destination.

### <Fourth Example Embodiment>

A fourth example embodiment is an improvement example of the aforementioned second example embodiment. In comparison with the aforementioned endoscope insertion assistance system 2000, the endoscope insertion assistance apparatus 30 is replaced by an endoscope insertion assistance apparatus 30b in an endoscope insertion assistance system according to the fourth example embodiment. Thus, illustrations thereof will be omitted and the following description will be focused on the changed parts.

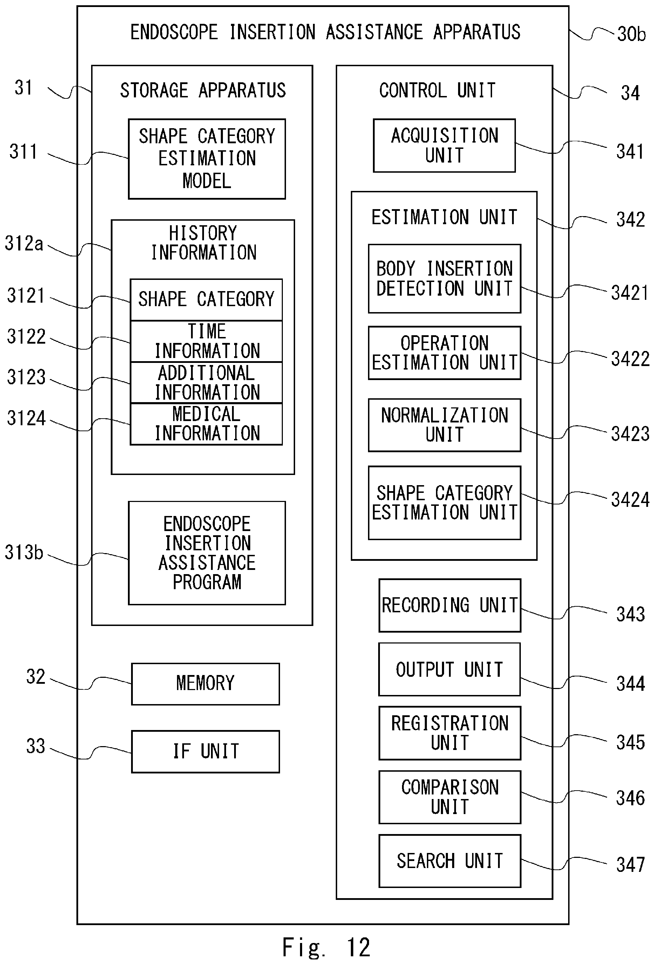

Fig. 12 is a block diagram illustrating a configuration of the endoscope insertion assistance apparatus 30b according to the fourth example embodiment. In comparison with the aforementioned endoscope insertion assistance apparatus 30a, the history information 312 is replaced by history information 312a, the endoscope insertion assistance program 313a is replaced by an endoscope insertion assistance program 313b and a search unit 347 is added in the endoscope insertion assistance apparatus 30b. Furthermore, medical information 3124 is added to the history information 312a. Note that, of the control unit 34, the endoscope insertion assistance apparatus 30b needs only to include at least the acquisition unit 341, the shape category estimation unit 3424, the recording unit 343, the output unit 344 and the search unit 347, and may not include the other components. The endoscope insertion assistance program 313b is a computer program in which a search process is implemented in the endoscope insertion assistance method according to the present example embodiment.

The search unit 347 searches for the history information 312 based on transition information indicating transition in chronological order of the estimated shape category, and outputs the search result. It is thereby possible to browse past history information similar to specific transition information. Thus, besides the transition information, it is possible to confirm differences in the additional information or the like and promote examinations. Note that the output by the search unit 347 may also be assumed to be output using the output unit 344. That is, the search unit 347 searches for the history information 312 using the transition information as a search condition and outputs the search result to the output unit 344, and the output unit 344 indicates that the search result is output to the display apparatus 41 or the speaker 42.

In the history information 312a, medical information 3124 is further associated with the shape category 3121, the time information 3122 and the additional information 3123. Here, the medical information 3124 is information indicating body information, clinical history or consultation history or the like of an examinee. Therefore, the search unit 347 may search for the history information 312 based on the medical information 3124 and output the search result. In this way, before carrying out an examination, it is possible to acquire and confirm past history information of the examinee similar to the medical information of the examinee this time. Therefore, it is possible to assist insertion of the endoscope into a patient having similar body information or clinical history by a more appropriate operation.

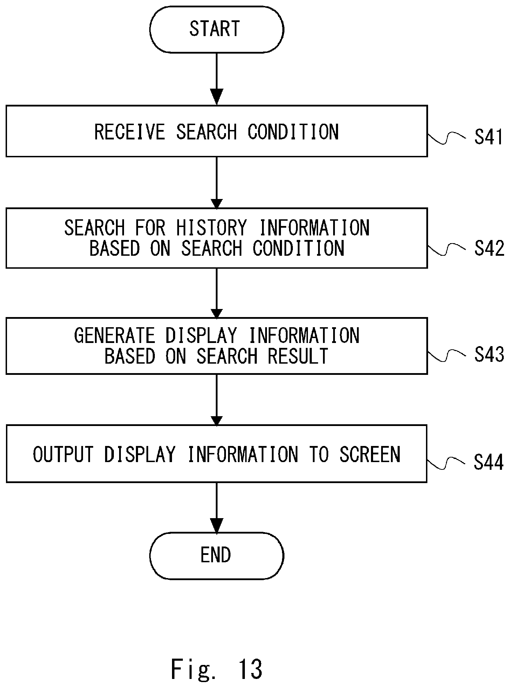

Fig. 13 is a flowchart illustrating a flow of a search process according to the fourth example embodiment. First, the search unit 347 receives a search condition of transition information (S41). For example, the input apparatus 43 receives inputs of date and time (examination time zone) or medical information corresponding to past endoscopies in accordance with operation by the medical doctors. The input apparatus 43 transmits the received date and time information or medical information to the endoscope insertion assistance apparatus 30b. The search unit 347 of the endoscope insertion assistance apparatus 30b retains the received date and time information or medical information in the memory 32 as a search condition.

Next, the search unit 347 searches for the history information 312 based on the search condition (S42). For example, when the search condition is date and time information, the search unit 347 acquires a set of the shape category 3121 associated with the time information 3122 included in a time zone indicated by the date and time information from the storage apparatus 31. For example, when the search condition is medical information, the search unit 347 acquires a set of the shape category 3121 associated with the medical information 3124 from the storage apparatus 31.

The search unit 347 generates display information based on the search result (S43). For example, regarding the transition information which is the search result, the search unit 347 generates the transition information as display information, as in the case of aforementioned step S209.

After that, the search unit 347 outputs the generated display information to the screen of the display apparatus 41 via the IF unit 33 (S44).

Thus, the present example embodiment can further promote an improvement of the endoscope insertion technique by effectively using the accumulated history information 312 of the second example embodiment.

### <Other Example Embodiments>

Note that the aforementioned example embodiments are applicable to examinations by the endoscope on the body cavity such as large intestine, small intestine, stomach or bronchus (bronchi) (lung).

Note that although the present disclosure has been described as a hardware configuration in the aforementioned example embodiments, the present disclosure is not limited to this. According to the present disclosure, arbitrary processes can be implemented by causing the CPU to execute a computer program.

In the above-described examples, the program can be stored in and supplied to a computer using various types of non-transitory computer-readable medium. The non-transitory computer-readable medium includes various types of tangible recording medium. Examples of the non-transitory computer-readable medium include magnetic recording medium (e.g., flexible disk, magnetic tape, hard disk drive), magnetooptical recording medium (e.g., magnetooptical disk), CD-ROM (read only memory), CD-R, CD-R/W, DVD (digital versatile disc), semiconductor memory (e.g., mask ROM, PROM (programmable ROM), EPROM (erasable PROM), flash ROM, RAM (random access memory)). The program may be supplied to the computer using various types of transitory computer-readable medium. Examples of the transitory computer-readable medium include electric signal, optical signal, and electromagnetic wave. The transitory computer-readable medium can supply the program to the computer through wired communication channels or wireless communication channels such as electric wires and optical fibers.

Note that the present disclosure is not limited to the above-described example embodiments, but can be changed as appropriate without departing from the spirit of the present disclosure. The present disclosure may be implemented by combining the respective example embodiments as appropriate.

Although some or all of the aforementioned example embodiments can also be described as the following supplementary notes, the present invention is not limited to the following supplementary notes.

### (Supplementary note A1)

An endoscope insertion assistance apparatus comprising:
acquisition means for acquiring shape data to identify an insertion shape of an endoscope inserted into a lumen;
estimation means for estimating any one of a plurality of shape categories in the insertion shape from the shape data; and
output means for outputting display information in the estimated shape category in chronological order.

### (Supplementary note A2)

The endoscope insertion assistance apparatus according to claim 1, wherein the output means outputs the display information indicating transition between the plurality of the estimated shape categories in the chronological order.

### (Supplementary note A3)

The endoscope insertion assistance apparatus according to claim 1 or 2, wherein the output means further outputs speech corresponding to the estimated shape category.

### (Supplementary note A4)

The endoscope insertion assistance apparatus according to any one of claims 1 to 3, wherein
the acquisition means further acquires an endoscope image captured by the endoscope, and
the estimation means estimates any one of the plurality of shape categories further using the endoscope image.

### (Supplementary note A5)

The endoscope insertion assistance apparatus according to claim 4, wherein
the estimation means estimates operation contents of the endoscope in the lumen based on a change of the endoscope image and the shape data, and
estimates any one of the plurality of shape categories with the operation contents of the endoscope further taken into account.

### (Supplementary note A6)

The endoscope insertion assistance apparatus according to claim 4 or 5, wherein
the estimation means identifies an examination starting point based on the endoscope image,
normalizes the shape data based on the examination starting point, and
estimates any one of the plurality of shape categories for the normalized shape data.

### (Supplementary note A7)

The endoscope insertion assistance apparatus according to any one of claims 4 to 6, further comprising recording means for identifying an examination starting time point based on the endoscope image and recording transition information in chronological order for the estimated shape category based on the examination starting time point.

### (Supplementary note A8)

The endoscope insertion assistance apparatus according to any one of claims 1 to 7, wherein the estimation means normalizes the shape data based on a posture detected from an examinee and estimates any one of the plurality of shape categories for the normalized shape data.

### (Supplementary note A9)

The endoscope insertion assistance apparatus according to any one of claims 1 to 8, further comprising registration means for receiving input of additional information on the estimated shape category and registering the additional information in association with the shape category.

### (Supplementary note A10)

The endoscope insertion assistance apparatus according to any one of claims 1 to 9, further comprising storage means for storing history information in which the estimated shape category is associated with time information.

### (Supplementary note A11)

The endoscope insertion assistance apparatus according to claim 10, further comprising comparison means for outputting a comparison result of two or more pieces of the history information.

### (Supplementary note A12)

The endoscope insertion assistance apparatus according to claim 11, wherein
the comparison means evaluates a comparison destination against a comparison source of the history information, and
outputs the evaluation result as the comparison result.

### (Supplementary note A13)

The endoscope insertion assistance apparatus according to claim 11 or 12, wherein
when the comparison result shows that a duration of a specific shape category is a predetermined time or more, the comparison means outputs a warning.

### (Supplementary note A14)

The endoscope insertion assistance apparatus according to any one of claims 10 to 13, further comprising search means for searching for the history information based on transition information indicating transition of the estimated shape categories in chronological order, and outputting a search result.

### (Supplementary note A15)

The endoscope insertion assistance apparatus according to claim 14, wherein
the history information is further associated with medical information on examinees, and
the search means searches for the history information based on the medical information and outputs the search result.

### (Supplementary note A16)

The endoscope insertion assistance apparatus according to any one of claims 1 to 15, wherein
the estimation means identifies a location in a body from the estimated shape category, and
the output means further outputs the identified location.

### (Supplementary note A17)

The endoscope insertion assistance apparatus according to any one of claims 1 to 16, wherein
the estimation means estimates any one of the plurality of shape categories from the acquired shape data using a learned model learned from the learning data labeled with the shape category on each of the plurality of pieces of shape data.

### (Supplementary note B1)

An endoscope insertion assistance method for causing a computer to:
acquire shape data to identify an insertion shape of an endoscope inserted into a lumen;
estimate any one of a plurality of shape categories in the insertion shape from the shape data; and
output display information in chronological order for the estimated shape category.

### (Supplementary note C1)

A non-transitory computer-readable medium storing an endoscope insertion assistance program for causing a computer to execute:
an acquisition process to acquire shape data to identify an insertion shape of an endoscope inserted into a lumen;
an estimation process to estimate any one of a plurality of shape categories in the insertion shape from the shape data; and
an output process to output display information in chronological order for the estimated shape category.

Although the present invention has been described with reference to the example embodiments (and the examples) so far, the present invention is not limited to the above-described example embodiments (and examples). Various changes can be made to the configuration and details of the present invention without departing from the scope of the present invention in a way understandable to those skilled in the art.

The present application claims a priority based on Japanese Patent Application No. 2020-070370, filed on April 9, 2020, the disclosure of which is incorporated herein by reference in its entirety.

### Reference Signs List

- 100: ENDOSCOPE INSERTION ASSISTANCE APPARATUS
- 110: ACQUISITION UNIT
- 120: ESTIMATION UNIT
- 130: OUTPUT UNIT
- 2000: ENDOSCOPE INSERTION ASSISTANCE SYSTEM
- 10: ENDOSCOPE APPARATUS
- 11: ELECTRONIC ENDOSCOPE
- 11a: INSERTION PORTION
- 20: ENDOSCOPE INSERTION SHAPE OBSERVATION APPARATUS
- 21: SENSE COIL UNIT
- 22: SHAPE PROCESSING APPARATUS
- 30: ENDOSCOPE INSERTION ASSISTANCE APPARATUS
- 30a: ENDOSCOPE INSERTION ASSISTANCE APPARATUS
- 30b: ENDOSCOPE INSERTION ASSISTANCE APPARATUS
- 31: STORAGE APPARATUS
- 311: SHAPE CATEGORY ESTIMATION MODEL
- 312: HISTORY INFORMATION
- 312a: HISTORY INFORMATION
- 3121: SHAPE CATEGORY
- 3122: TIME INFORMATION
- 3123: ADDITIONAL INFORMATION
- 3124: MEDICAL INFORMATION
- 313: ENDOSCOPE INSERTION ASSISTANCE PROGRAM
- 313a: ENDOSCOPE INSERTION ASSISTANCE PROGRAM
- 313b: ENDOSCOPE INSERTION ASSISTANCE PROGRAM
- 314: SHAPE DATA
- 315: ENDOSCOPE IMAGE
- 316: DISPLAY INFORMATION
- 316a: COMPARISON RESULT
- 32: MEMORY
- 33: IF UNIT
- 34: CONTROL UNIT
- 341: ACQUISITION UNIT
- 342: ESTIMATION UNIT
- 3421: BODY INSERTION DETECTION UNIT
- 3422: OPERATION ESTIMATION UNIT
- 3423: NORMALIZATION UNIT
- 3424: SHAPE CATEGORY ESTIMATION UNIT
- 343: RECORDING UNIT
- 344: OUTPUT UNIT
- 345: REGISTRATION UNIT
- 346: COMPARISON UNIT
- 347: SEARCH UNIT
- 41: DISPLAY APPARATUS
- 42: SPEAKER
- 43: INPUT APPARATUS
- U1: MEDICAL DOCTOR
- U2: EXAMINEE
- C1: SHAPE CATEGORY
- C11: SHAPE SUBCATEGORY
- C12: SHAPE SUBCATEGORY
- C13: SHAPE SUBCATEGORY
- C2: SHAPE CATEGORY
- C21: SHAPE SUBCATEGORY
- C22: SHAPE SUBCATEGORY
- C3: SHAPE CATEGORY
- C31: SHAPE SUBCATEGORY
- C32: SHAPE SUBCATEGORY
- C33: SHAPE SUBCATEGORY
- C4: SHAPE CATEGORY
- C41: SHAPE SUBCATEGORY

## Claims

1. An endoscope insertion assistance apparatus comprising:
acquisition means for acquiring shape data to identify an insertion shape of an endoscope inserted into a lumen;
estimation means for estimating any one of a plurality of shape categories in the insertion shape from the shape data; and
output means for outputting display information in the estimated shape category in chronological order.

2. The endoscope insertion assistance apparatus according to claim 1, wherein the output means outputs the display information indicating transition between the plurality of the estimated shape categories in the chronological order.

3. The endoscope insertion assistance apparatus according to claim 1 or 2, wherein the output means further outputs speech corresponding to the estimated shape category.

4. The endoscope insertion assistance apparatus according to any one of claims 1 to 3, wherein
the acquisition means further acquires an endoscope image captured by the endoscope, and
the estimation means estimates any one of the plurality of shape categories further using the endoscope image.

5. The endoscope insertion assistance apparatus according to claim 4, wherein
the estimation means estimates operation contents of the endoscope in the lumen based on a change of the endoscope image and the shape data, and
estimates any one of the plurality of shape categories with the operation contents of the endoscope further taken into account.

6. The endoscope insertion assistance apparatus according to claim 4 or 5, wherein
the estimation means identifies an examination starting point based on the endoscope image,
normalizes the shape data based on the examination starting point, and
estimates any one of the plurality of shape categories for the normalized shape data.

7. The endoscope insertion assistance apparatus according to any one of claims 4 to 6, further comprising recording means for identifying an examination starting time point based on the endoscope image and recording transition information in chronological order for the estimated shape category based on the examination starting time point.

8. The endoscope insertion assistance apparatus according to any one of claims 1 to 7, wherein the estimation means normalizes the shape data based on a posture detected from an examinee and estimates any one of the plurality of shape categories for the normalized shape data.

9. The endoscope insertion assistance apparatus according to any one of claims 1 to 8, further comprising registration means for receiving input of additional information on the estimated shape category and registering the additional information in association with the shape category.

10. The endoscope insertion assistance apparatus according to any one of claims 1 to 9, further comprising storage means for storing history information in which the estimated shape category is associated with time information.

11. The endoscope insertion assistance apparatus according to claim 10, further comprising comparison means for outputting a comparison result of two or more pieces of the history information.

12. The endoscope insertion assistance apparatus according to claim 11, wherein
the comparison means evaluates a comparison destination against a comparison source of the history information, and
outputs the evaluation result as the comparison result.

13. The endoscope insertion assistance apparatus according to claim 11 or 12, wherein
when the comparison result shows that a duration of a specific shape category is a predetermined time or more, the comparison means outputs a warning.

14. The endoscope insertion assistance apparatus according to any one of claims 10 to 13, further comprising search means for searching for the history information based on transition information indicating transition of the estimated shape categories in chronological order, and outputting a search result.

15. The endoscope insertion assistance apparatus according to claim 14, wherein
the history information is further associated with medical information on examinees, and
the search means searches for the history information based on the medical information and outputs the search result.

16. The endoscope insertion assistance apparatus according to any one of claims 1 to 15, wherein
the estimation means identifies a location in a body from the estimated shape category, and
the output means further outputs the identified location.

17. The endoscope insertion assistance apparatus according to any one of claims 1 to 16, wherein
the estimation means estimates any one of the plurality of shape categories from the acquired shape data using a learned model learned from the learning data labeled with the shape category on each of the plurality of pieces of shape data.

18. An endoscope insertion assistance method for causing a computer to:
acquire shape data to identify an insertion shape of an endoscope inserted into a lumen;
estimate any one of a plurality of shape categories in the insertion shape from the shape data; and
output display information in chronological order for the estimated shape category.

19. A non-transitory computer-readable medium storing an endoscope insertion assistance program for causing a computer to execute:
an acquisition process to acquire shape data to identify an insertion shape of an endoscope inserted into a lumen;
an estimation process to estimate any one of a plurality of shape categories in the insertion shape from the shape data; and
an output process to output display information in chronological order for the estimated shape category.
